# NEW EUROPEAN PATENT SPECIFICATION

(11) **EP 2 773 760 B2**
(45) Date of publication and mention of the opposition decision: **04.11.2020**
(45) Mention of the grant of the patent: 06.12.2017
(21) Application number: 12778763.8
(22) Date of filing: 31.10.2012
(51) Int. Cl.: C12N 15/117, A61K 31/713, A61K 39/39

(54) **DOUBLE-STRANDED RNA FOR IMMUNOSTIMULATION**
DOPPELSTRÄNGIGE RNA ZUR IMMUNSTIMULATION
ARN DOUBLE BRIN POUR L'IMMUNOSTIMULATION

(30) Priority: 31.10.2011 EP 11187345; 30.12.2011 EP 11196165; 30.12.2011 US 201161581983 P
(43) Date of publication of application: 10.09.2014
(73) Proprietor: RiboxX GmbH, 01445 Radebeul (DE)
(72) Inventor: ROHAYEM, Jacques, 01277 Dresden (DE)
(74) Representative: Habermann, Hruschka & Schnabel
(86) International application number: PCT/EP2012/071640
(87) International publication number: WO 2013/064584

(56) References cited:
- EP-A1- 2 123 757
- WO-A1-2010/097414
- WO-A1-2011/034950
- WO-A2-2008/014979
- WO-A2-2009/095226
- WO-A2-2012/027017
- LU C ET AL: "The Structural Basis of 5' Triphosphate Double-Stranded RNA Recognition by RIG-I C-Terminal Domain", STRUCTURE, vol. 18, no. 8, 11 August 2010 (2010-08-11), pages 1032-1043, XP027196954, ISSN: 0969-2126

### SEQUENCE LISTING

<110> RiboxX GmbH
<120> Double-stranded RNA for immunostimulation
<130> R 0117 WO
<150> EP11187345.1
   <151> 2011-10-31
<150> EP11196165.2
   <151> 2011-12-30
<150> US61/581,983
   <151> 2011-12-30
<160> 15
<170> BiSSAP 1.0
<210> 1
   <211> 45
   <212> RNA
   <213> artificial sequences
<220>
   <221> source
   <222> 1..45
   <223> /mol_type="RNA" /note="Prior art RNA" /organism="artificial sequences"
<400> 1
   gggggggguu uuuuuuuuuu uuuuuuuuuu uuuuuuuugg ggggg 45
<210> 2
   <211> 47
   <212> RNA
   <213> artificial sequences
<220>
   <221> source
   <222> 1..47
   <223> /mol_type="RNA" /note="Prior art RNA" /organism="artificial sequences"
<400> 2
   gggggggggu uuuuuuuuuu uuuuuuuuuu uuuuuuuuug ggggggg 47
<210> 3
   <211> 57
   <212> RNA
   <213> artificial sequences
<220>
   <221> source
   <222> 1..57
   <223> /mol_type="RNA" /note="Prior art RNA" /organism="artificial sequences"
<400> 3
   ggguuuuuuu uuuuuuuugg guuuuuuuuu uuuuuugggu uuuuuuuuuu uuuuggg 57
<210> 4
   <211> 51
   <212> RNA
   <213> artificial sequences
<220>
   <221> source
   <222> 1..51
   <223> /mol_type="RNA" /note="Prior art RNA" /organism="artificial sequences"
<400> 4
   ggguuugggu uuggguuugg guuuggguuu ggguuugggu uuggguuugg g 51
<210> 5
   <211> 57
   <212> RNA
   <213> artificial sequences
<220>
   <221> source
   <222> 1..57
   <223> /mol_type="RNA" /note="Prior art RNA" /organism="artificial sequences"
<400> 5
   cccuuuuuuu uuuuuuuucc cuuuuuuuuu uuuuuucccu uuuuuuuuuu uuuuccc 57
<210> 6
   <211> 51
   <212> RNA
   <213> artificial sequences
<220>
   <221> source
   <222> 1..51
   <223> /mol_type="RNA" /note="Prior art RNA" /organism="artificial sequences"
<400> 6
   cccuuucccu uucccuuucc cuuucccuuu cccuuucccu uucccuuucc c 51
<210> 7
   <211> 40
   <212> RNA
   <213> artificial sequences
<220>
   <221> source
   <222> 1..40
   <223> /mol_type="RNA" /note="Prior art RNA" /organism="artificial sequences"
<400> 7
   cccccccccc cccccccccc gguuuuuuuu uuuuuuuggg 40
<210> 8
   <211> 48
   <212> RNA
   <213> artificial sequences
<220>
   <221> source
   <222> 1..48
   <223> /mol_type="RNA" /note="Riboxxim-1 sense strand" /organism="artificial sequences"
<400> 8
   cccccuaagc acgaagcuca gaguuaagca cgaagcucag aguccccc 48
<210> 9
   <211> 48
   <212> RNA
   <213> artificial sequences
<220>
   <221> source
   <222> 1..48
   <223> /mol_type="RNA" /note="Riboxxim-1/Riboxxim-48 antisense strand" /organism="artificial sequences"
<400> 9
   gggggacucu gagcuucgug cuuaacucug agcuucgugc uuaggggg 48
<210> 10
   <211> 46
   <212> RNA
   <213> artificial sequences
<220>
   <221> source
   <222> 1..46
   <223> /mol_type="RNA" /note="Riboxxim-2 sense strand" /organism="artificial sequences"
<400> 10
   cccccgaacg aauuuauaag ugggaacgaa uuuauaagug gccccc 46
<210> 11
   <211> 46
   <212> RNA
   <213> artificial sequences
<220>
   <221> source
   <222> 1..46
   <223> /mol_type="RNA" /note="Riboxxim-2 antisense strand" /organism="artificial sequences"
<400> 11
   gggggccacu uauaaauucg uucccacuua uaaauucguu cggggg 46
<210> 12
   <211> 46
   <212> RNA
   <213> artificial sequences
<220>
   <221> source
   <222> 1..46
   <223> /mol_type="RNA" /note="Riboxxim-3 sense strand" /organism="artificial sequences"
<400> 12
   cccccacaac auucauauag cugacaacau ucauauagcu gccccc 46
<210> 13
   <211> 46
   <212> RNA
   <213> artificial sequences
<220>
   <221> source
   <222> 1..46
   <223> /mol_type="RNA" /note="Riboxxim-3 antisense strand" /organism="artificial sequences"
<400> 13
   gggggcagcu auaugaaugu ugucagcuau augaauguug uggggg 46
<210> 14
   <211> 90
   <212> RNA
   <213> artificial sequences
<220>
   <221> source
   <222> 1..90
   <223> /mol_type="RNA" /note="dsRNA-90/Riboxxim-90 sense strand" /organism="artificial sequences"
<400> 14
   cccccuaagc agcaagccuc agcagcuaag ccagcagccu cagcagcuag cagcaagcuc    60
   agcagcuaag ccacgagcuc augcgccccc                                     90
<210> 15
   <211> 90
   <212> RNA
   <213> artificial sequences
<220>
   <221> source
   <222> 1..90
   <223> /mol_type="RNA" /note="dsRNA-90/Riboxxim-90 antisense strand" /organism="artificial sequences"
<400> 15
   gggggcgcau gagcucgugg cuuagcugcu gagcuugcug cuagcugcug aggcugcugg    60
   cuuagcugcu gaggcuugcu gcuuaggggg                                     90

## Description

The present invention relates to a ribonucleic acid (RNA) of double-stranded structure as defined in claim 1, which RNA is capable of triggering Toll-like receptor 3 (TLR-3) and which shows an increased serum stability while simultaneously being unable to be processed by the DICER complex.

It is known that double-stranded RNA causes immunostimulation through the TLR-3 located in endosomes of dendritic and epithelial cells (see Kucnick et al. (2010) Current Medical Chemistry; Coffmann et al. (2010) Immunity).

WO 2008/014979 A2 and WO 2009/095226 A2 disclose nucleic acids of the general formula GₗXₘGₙ or CₗXₘCₙ and (NᵤGₗXₘGₙNᵥ)ₐ or (NᵤCₗXₘCₙNᵥ)ₐ, respectively. According to this prior art, such nucleic acids can be double-stranded RNA and have immunostimulative properties. However, specific examples of molecules according to the above formulae disclosed in WO 2008/014979 A2 and WO 2009/095226 A2 are single-stranded and have highly artificial sequences containing stretches of G or C nucleotides at both ends and a polyU stretch or a repeat containing C or G and U nucleotides between the C or G, respectively, ends such as the following:
GGGGGGGGUUUUUUUUUUUUUUUUUUUUUUUUUUUUUUGGGGGGG (SEQ ID NO: 1);
GGGGGGGGGUUUUUUUUUUUUUUUUUUUUUUUUUUUUUUGGGGGGGG (SEQ ID NO: 2);
GGGUUUUUUUUUUUUUUUGGGUUUUUUUUUUUUUUUGGGUUUUUUUUUUUUUUUGGG (SEQ ID NO: 3);
GGGUUUGGGUUUGGGUUUGGGUUUGGGUUUGGGUUUGGGUUUGGGUUUGGG (SEQ ID NO: 4);
CCCUUUUUUUUUUUUUUUCCCUUUUUUUUUUUUUUUCCCUUUUUUUUUUUUUUUCCC (SEQ ID NO: 5);
CCCUUUCCCUUUCCCUUUCCCUUUCCCUUUCCCUUUCCCUUUCCCUUUCCC (SEQ ID NO: 6); and
CCCCCCCCCCCCCCCCCCCCGGUUUUUUUUUUUUUUUGGG (SEQ ID NO: 7).

WO 2010/097414 A1 describes small-interfering RNA molecules displaying an increasedd thermodynamic stability at the 3' end of the antisense strand and the 5' end oft he sense strand in comparison to the base pairing in the seed region.

The technical problem underlying the present invention is to provide an improved system for immunostimulation using RNA molecules.

The solution to the above technical problem is provided by the embodiments as defined in claims 1 to 16.

In particular, the present invention provides a ribonucleic acid as defined in claim 1, which ribonucleic acid has at least a segment of double-strand structure of a minimum of 45 bp wherein said double-stranded segment has increased thermodynamic stability on both ends (or end regions) of the dsRNA or dsRNA segment, respectively.

More particularly, it is provided a ribonucleic acid as defined in claim 1 having a double-stranded structure of at least 45 bp, preferably of at least 48 bp, more preferably of at least 70 bp, particularly preferred of from 45, 46, 47 or 48 to 200 bp, wherein said double-stranded structure has a first and a second end each having 5 consecutive G/C bp at the respective end of the double-stranded structure. Due to the increased presence of G/C base pairing at both ends of the double-stranded structure it results a thermodynamic "clamp" providing the RNA of the invention with a surprisingly high serum-stability and stability against cellular RNA degrading enzymes such as DICER. Accordingly, the inventive RNAs cannot be processed by cellular RNAses like the DICER complex. Therefore, the ribonucleic acid of the present invention displays a superior triggering of the TLR-3 pathway leading to a sustained and powerful immunostimulation (innate immune response).

The ribonucleic acid according to the invention has a nucleotide sequence between the last 6 to 20 bp at each end that is heteropolymeric, wherein ribonucleic acids having the following sequences are excluded:
GGGUUUUUUUUUUUUUUUGGGUUUUUUUUUUUUUUUGGGUUUUUUUUUUUUUUUGGG (SEQ ID NO: 3)
GGGUUUGGGUUUGGGUUUGGGUUUGGGUUUGGGUUUGGGUUUGGGUUUGGG (SEQ ID NO: 4)
CCCUUUUUUUUUUUUUUUCCCUUUUUUUUUUUUUUUCCCUUUUUUUUUUUUUUUCCC (SEQ ID NO: 5)
CCCUUUCCCUUUCCCUUUCCCUUUCCCUUUCCCUUUCCCUUUCCCUUUCCC (SEQ ID NO: 6)

As defined in claim 1 the 5 terminal bp of each end of the double-stranded structure in the ribonucleic acid of the invention are G/C bp.

It is not necessary that the ribonucleic acid of the present invention is completely double-stranded. However, it is essentially double-stranded, i.e. there is regular hydrogen bonding between the opposite bases of the strands forming the double-stranded structure, but there may be one or several mismatches through non-complementary bases or through chemical modifications. Usually, such mismatches do not exceed 10%, more preferably 5 %, particularly preferred 2% or even 1% of the double-stranded molecule (or double-stranded segment of the inventive RNA, respectively).

According to preferred embodiments, the double-stranded ribonucleic acid of the invention may also have overhangs at one or both ends of the double-stranded structure which can be of various length but typically do not exceed ten single-stranded residues, more preferably such overhangs comprise one, two or three residues. These overhangs may also comprise deoxyribonucleotides instead of ribonucleotides.

The ribonucleic acid according to the present invention comprises two separate polyribonucleotide strands.

As defined in claim 1 the strands of the at least one segment of double-stranded structure are one of the following sequence pairs:
5'-(G)₅(N)_{y}(G)₅-3'
3'-(C)₅(N)_{y}(C)₅-5'
or
5'-(C)₅(N)_{y}(C)₅-3'
3'-(G)₅(N)_{y}(G)₅-5'
or
5'-(G)₅(N)_{y}(C)₅-3'
3'-(C)₅(N)_{y}(G)₅-5'
or
5'-(C)₅(N)_{y}(G)₅-3'
3'-(G)₅(N)_{y}(C)₅-5'
wherein y is typically an integer of from 35 to 200 or more such as 500 or 600, preferably 35 to 150, more preferably 35 to 100, particularly preferred 35 to 80, even more preferred 35 to 40.

The sequence of the double-stranded region between the G/C "clamps" of the ribonucleic acid of the invention is in principle not critical, however, homopolymers of any of the ribonucleobases A, U, G, or C, in particular G or C, are not well accepted due to their potential toxicity. Normally, the sequence is chosen such that the overall sequence shows no evident complementarities to other existing RNA or DNA species in a cell or organism, particularly in order to avoid potential side effects from posttranscriptional gene silencing mechanisms. However, it may be appropriate that the sequence between the C/C base pairs at both ends, or generally speaking, between the G/C clamps as defined above, may be assembled from naturally occurring (partial) sequences that may be repeated. An example is a certain (partial) sequence of, e.g. 20 to 90 nucleotides, of a naturally occurring sequence, e.g. of a virus, microorganism or other organism, that is repeated once or several times such as two, three, four or five or even more times, in order to provide a molecule of the present invention having the appropriate or desired, respectively, length.

In contrast thereto, with regard to an easier and less expensive production and also in order to avoid highly artificial and potentially toxic sequences such as those disclosed in WO 2008/014979 and WO 2009/095226, it is preferred that the double-stranded region between the G/C clamps, is free of homopolymeric stretches of more than 5 contiguous identical nucleotides, and is free of short repeated sequence motifs of 3 to 10 nucleotides per motif.

Furthermore, in order to further increase the stability of the RNA of the invention, the G/C content of the double-stranded structure (comprising the G/C clamps) is at least 45%, more preferably 45 to 70%, particularly preferred 48 to 60%. It is also contemplated that the sequence between the "clamps" contains further (short) stretches of G/C base pairs, e.g. 3, 4 or 5 G/C base pairs.

Such embodiments of the invention usually have a Tm of the double-stranded structure of from 68 to 80 C.

In consideration of production costs to effective triggering of TLR-3 (dimerization), preferred lengths of the dsRNA structure, more preferably of the complete RNA, range from 45 to 150 bp, particularly preferred from 45 to 100 bp, even more preferred from 45 to 50 bp, i.e. 45, 46, 47, 48, 49 or 50 bp.

Specific preferred examples of the double-stranded RNA according to the invention are composed of the following (complementary) sequence pairs:
Riboxxim®-1:
   5'-CCCCCUAAGCACGAAGCUCAGAGUUAAGCACGAAGCUCAGAGUCCCCC-3' (SEQ ID NO: 8)
   5'-GGGGGACUCUGAGCUUCGUGCUUAACUCUGAGCUUCGUGCUUAGGGGG-3' (SEQ ID NO: 9)
Riboxxim®-2:
   5'-CCCCCGAACGAAUUUAUAAGUGGGAACGAAUUUAUAAGUGGCCCCC-3' (SEQ ID NO: 10)
   5'-GGGGGCCACUUAUAAAUUCGUUCCCACUUAUAAAUUCGUUCGGGGG-3' (SEQ ID NO: 11)
Riboxxim®-3:
   5'-CCCCCACAACAUUCAUAUAGCUGACAACAUUCAUAUAGCUGCCCCC-3' (SEQ ID NO: 12)
   5'-GGGGGCAGCUAUAUGAAUGUUGUCAGCUAUAUGAAUGUUGUGGGGG-3' (SEQ ID NO: 13)
dsRNA-90:

As mentioned before, the RNA of the present invention may have overhangs on one or both sides, but it is typically blunt-ended on both sides of the double-strand structure.

It is contemplated that the RNA of the present invention is equipped with further entities to improve the immunostimulative properties. For example, it would be possible to include CpG deoxyribonucleotide motifs into the ribonucleic acid of the invention (one or more of them) in order to trigger the Toll-like receptor 9 (TRL-9), at least partially.

Further particularly preferred ribonucleic acids of the invention have a free triphosphate group at the 5'-end of at least one strand. Such triphosphate-containing RNAs of the invention are preferably completely double-stranded and one or both 5' ends contain(s) a free triphosphate group. A "free triphosphate group" in this context means that this triphosphate group is not modified and specifically does not contain, or is not part of, respectively, a cap structure. Such triphosphate-containing RNAs of the invention are particularly potent immunostimulative compounds. Especially preferred examples of ribonucleic acids according to the invention containing a triphosphate group are the following sequence pairs (the complementary strands are given in 5' to 3' orientation):
Riboxxim®-48:
   5'-CCCCCUAAGCACGAAGCUCAGAGUUAAGCACGAAGCUCAGAGUCCCCC-3' (SEQ ID NO: 8)
   ppp-5'-GGGGGACUCUGAGCUUCGUGCUUAACUCUGAGCUUCGUGCUUAGGGGG-3' (SEQ ID NO: 9)
Riboxxim®-90:
wherein ppp denotes the free 5'-triphosphate group.

A free triphosphate-containing double-stranded ribonucleic acid of the present invention triggers both RIG-I (see. e.g. WO 2008/017473 A2) and TLR-3 pathways and exerts a dose-dependent innate immune response as demonstrated in the examples.

The RNA of the present invention may also contain one or more modified nucleotide analogues, in particular based upon stability considerations.

The chemical modification of the nucleotide analogue in comparison to the natural occurring nucleotide may be at the ribose, phosphate and/or base moiety. With respect to molecules having an increased stability, especially with respect to RNA degrading enzymes, modifications at the backbone, i. e. the ribose and/or phosphate moieties, are especially preferred.

Preferred examples of ribose-modified ribonucleotides are analogues wherein the 2'-OH group is replaced by a group selected from H, OR, R, halo, SH, SR, NH₂, NHR, NR₂, or CN with R being C₁-C₆ alkyl, alkenyl or alkynyl and halo being F, Cl, Br or I. It is clear for the person skilled in the art that the term "modified ribonucleotide" also includes 2'-deoxyderivatives, such as 2'-O-methyl derivatives, which may at several instances also be termed "deoxynucleotides".

As mentioned before, the at least one modified ribonucleotide may be selected from analogues having a chemical modification at the base moiety. Examples of such analogues include, but are not limited to, 5-aminoallyl-uridine, 6-aza-uridine, 8-aza-adenosine, 5-bromo-uridine, 7-deaza-adenine, 7-deaza-guanine, N⁶-methyl-adenine, 5-methyl-cytidine, pseudo-uridine, and 4-thio-uridine.

Examples of backbone-modified ribonucleotides wherein the phosphoester group between adjacent ribonucleotides is modified are phosphothioate groups.

The present invention also provides a method of producing the RNA as defined herein comprising the step of:
synthesizing a first ribonucleic acid strand and a second ribonucleic acid strand, and annealing the two strands under hybridization conditions wherein the strands have the appropriate sequences so as to form the double-stranded structure as defined herein.

The polynucleotide molecules for providing the double-stranded RNA of the present invention may be prepared by chemical synthesis methods or enzymatically, or by a combination of chemical and enzymatic steps. Methods for the enzymatic preparation of the double-stranded RNA molecules of the present invention are preferably those that use RNA-dependent RNA polymerases of caliciviruses as disclosed in WO-A-2007/012329. With respect to enzymatic synthesis of chemically modified dsRNAs using RNA-dependent RNA polymerases, the methods referred to in WO 2009/150156 are preferred. Chemical synthesis methods for preparing RNA strands are also well-known in the art.

The present invention also contemplates a vector encoding RNA species as defined herein. For example, a single-stranded dsRNA-containing entity as disclosed herein may be expressed in the form of a shRNA derivative.

Appropriately, double-stranded RNAs of the invention can also be labelled (for example, if there is an overhang on one side of the double-strand structure) for detection in research or diagnostic applications. The "label" can be any chemical entity which enables the detection of the RNA in question via physical, chemical and all biological means. Examples of typical labels linked to or integrated into one or more of the nucleotides or added to one end of the molecules as disclosed herein are radioactive labels, chromophores and fluorophores (e. g. fluorescein, TAM etc.).

The ribonucleic acids of the present invention are particularly for use as agonists of TLR-3. Further preferred ribonucleic acids of the invention containing a free 5'-triphosphate moiety are also agonists of RIG-I. In that function, the RNA molecules of the present invention exert an immunostimulatory effect in cells or organisms. Ribonucleic acids of the present invention are therefore useful as medicaments, in particular immunostimulatory preparations. Ribonucleic acids of the present invention are also contemplated for the manufacture of a medicament for immunostimulation. The present invention is therefore also directed to a pharmaceutical composition comprising at least one ribonucleic acid as defined herein in combination with at least one pharmaceutically acceptable carrier, excipient, and/or diluent. The preparation of pharmaceutical compositions in the context of the present invention, their dosages and their routes of administration are known to the skilled person, and guidance can be found in the latest edition of Remington's Pharmaceutical Sciences (Mack publishing Co., Eastern, PA, USA). It is also contemplated that the pharmaceutical compositions of the invention contain two or more different RNAs as defined herein.

In cases where the RNA of the invention is used as an immunostimulatory drug alone, a topical administration of the appropriate preparation (e.g. a spray) to skin and/or mucosa is preferred. As an "RNA drug" the RNA of the present invention leads to stimulation of dendritic cells and generation of a CD8+ T cell response. Such application of the inventive dsRNA is especially useful in the treatment of infectious diseases, e.g. by viruses (such as Herpesvirus, Papillomavirus) or in the treatment of cancer.

The improved immunostimulatory effects the ribonucleic acids of the present invention are also useful in combination with a vaccine directed to a certain disease. Thus, the RNA of the invention may also be used as an "RNA adjuvant" in vaccine preparations (or administered in a distinct preparation together with the vaccine, or sequentially). Simultaneous or sequential administration of the vaccine and the immunostimulatory RNA of the present invention should improve the immune response against the antigen of the vaccine by generating a protective CD8+ T cell response to soluble proteins (antigens), triggering DC activation and induction of type I IFN production.

The ribonucleic acids or pharmaceutical compositions as disclosed herein can also be combined with further immunostimulatory drugs known in the art.

The ribonucleic acids of the present invention are particularly useful in the treatment of diseases, including infectious diseases caused by infectious agents such as a bacterium, virus, and fungus, and tumours.

Also disclosed is a method for the treatment of a disease as mentioned above, preferably a viral infection or a tumour disease, comprising administering an effective amount of the pharmaceutical composition of the invention to a preferably mammalian, particularly human, patient in need of such treatment.

Also disclosed is a cell or non-human organism being transfected or transformed with the double-stranded RNA molecule as defined herein or with a vector coding therefor.

The figures show:

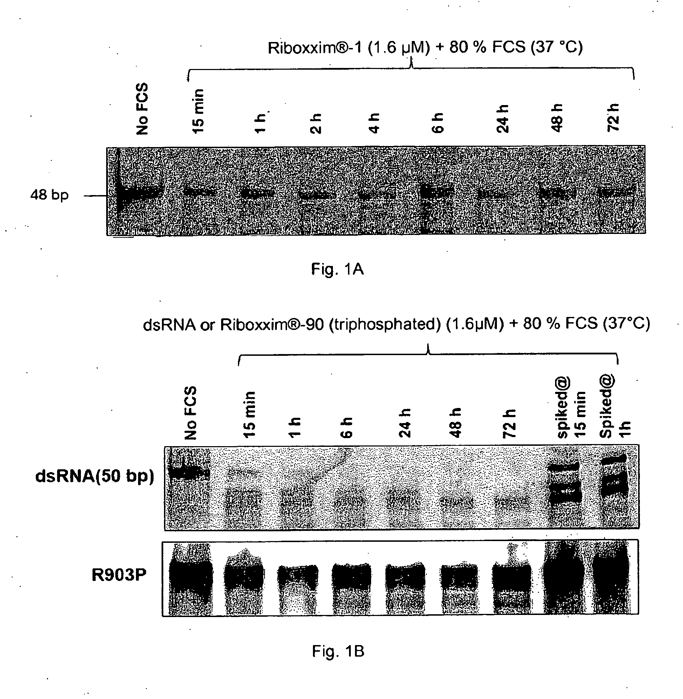

Fig. 1 shows a native polyacrylamide gel (20%) after ethidium bromide staining of reactions demonstrating that an exemplary ribonucleic acid of the invention (Riboxxim®-1) is stable for at least 72 h in 80% FCS at 37°C. A reaction containing no FCS was used as a negative control.

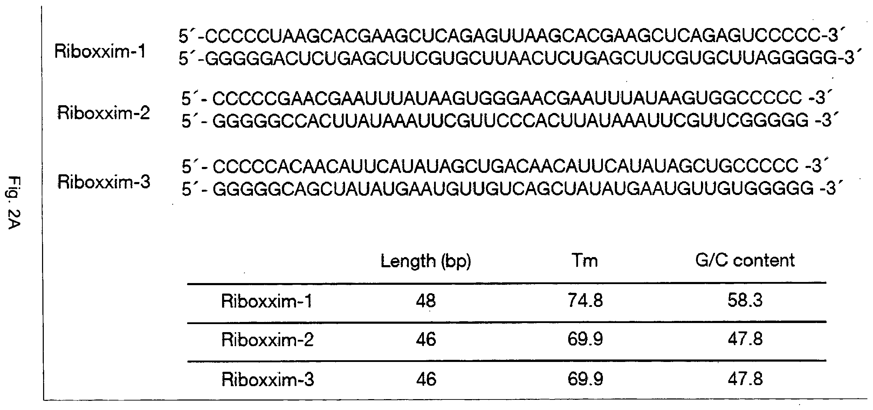

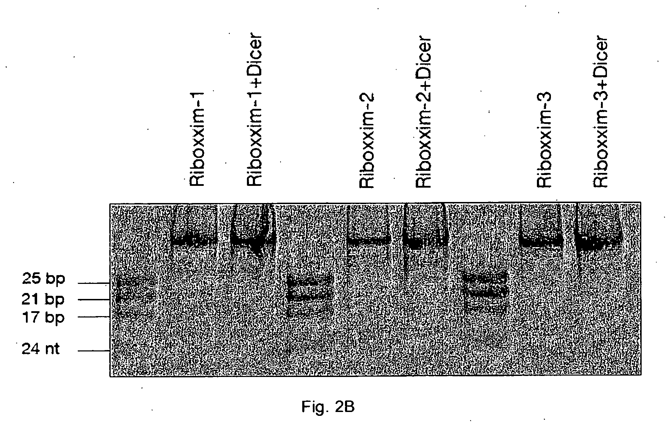

Fig. 2 shows that ribonucleic acids of the present invention are resistant to human DICER. (A) Sequences of the strands in the double-stranded molecules in the exemplary RNAs of the invention called Riboxxim®-1, Riboxxim®-2 and Riboxxim®-3: length (in base pairs), Tm and G/C content are indicated. (B) Native polyacrylamide gel (20%) after ethidium bromide staining of reactions containing 1.6 µM of Riboxxim®-1, -2 or -3 and recombinant human DICER incubated at 37 °C for 12 h. It can be seen that Riboxxim®-1 Riboxxim®-2 and Riboxxim®-3 are not degraded by human DICER. dsRNA marker of 17 bp, 21 bp and 25 bp, and a ssRNA marker of 24 nt are indicated.

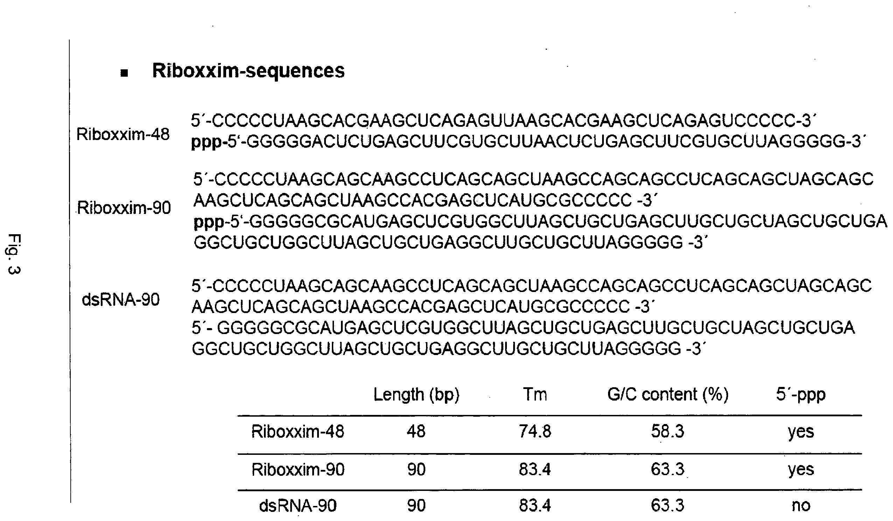

Fig. 3 shows the sequences of the strands of double-stranded RNA molecules with a triphosphate moiety at one 5' end according to the invention called Riboxxim®-48 and Riboxxim®-90 as well as the sequence of a double-stranded RNA molecule without a triphosphate moiety at one 5' end (dsRNA-90); length (in base pairs), Tm, G/C content and the presence of the triphosphate moiety are indicated.

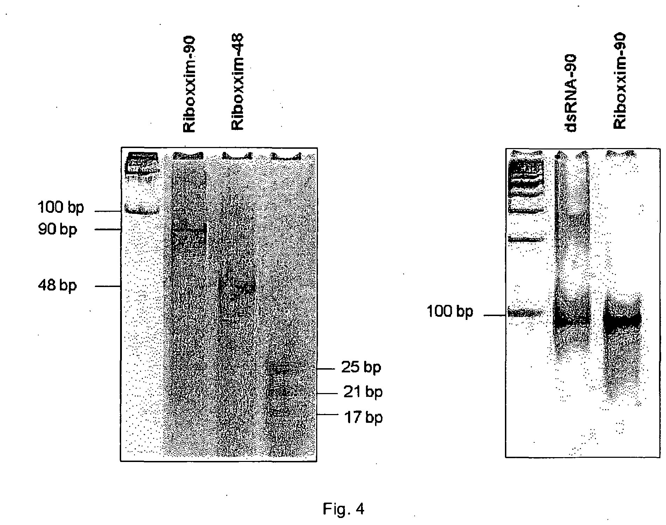

Fig. 4 shows a photograph of an ethidium bromide-stained 10% polyacrylamide gel after electrophoretic separation of the indicated nucleic acids demonstrating their length. Left panel: An RNA marker corresponding to dsRNA of 100 bp is shown in the first lane from the left, and an RNA marker corresponding to dsRNA of 25 bp, 21 bp and 17 bp is shown in the fourth lane from the left. RNAs according to the invention Riboxxim®-90 and Riboxxim® 48 are shown in the second and third lane, respectively, from the left. Right panel: the first lane from the left shows an RNA marker corresponding *inter alia* to dsRNA of 100 bp, as indicated. The second and third lane from the left show dsRNA-90 and Riboxxim®-90, respectively.

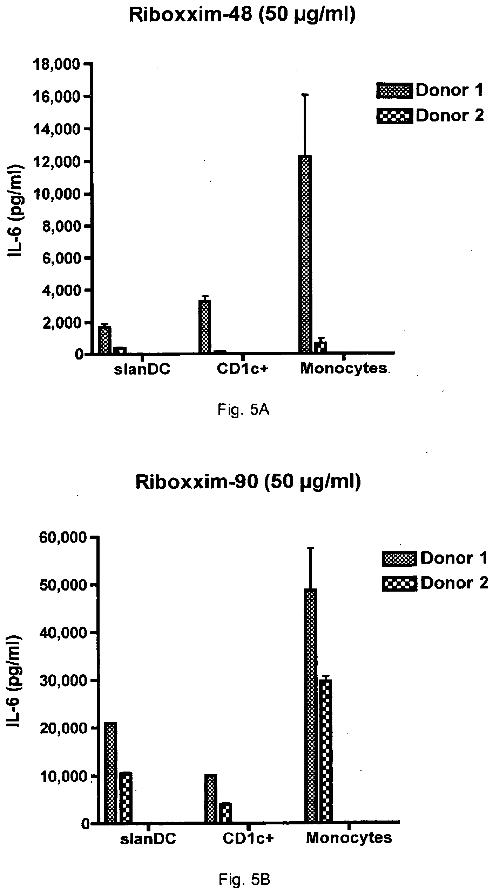

Fig. 5 shows two diagrams indicating the concentration of the cytokine IL-6 in the cell culture supernatant of dendritic cells (slanDC, CD1c+) and monocytes due to secretion of the cytokine by the indicated cells after stimulation with double-stranded RNA according to the invention bearing a triphosphate moiety, Riboxxim®-48 (Fig. 5A) or Riboxxim®-90 (Fig. 5B).

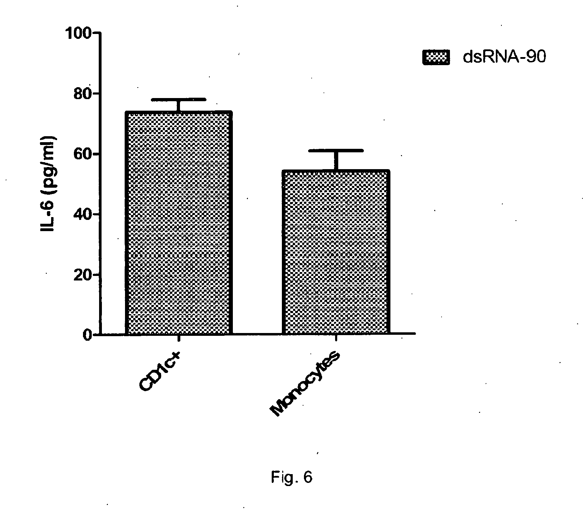

Fig. 6 shows a diagram indicating the amount of secreted IL-6 after stimulation of myeloid dendritic cells (CD1c+) and monocytes with a double-stranded RNA according to the invention (dsRNA-90).

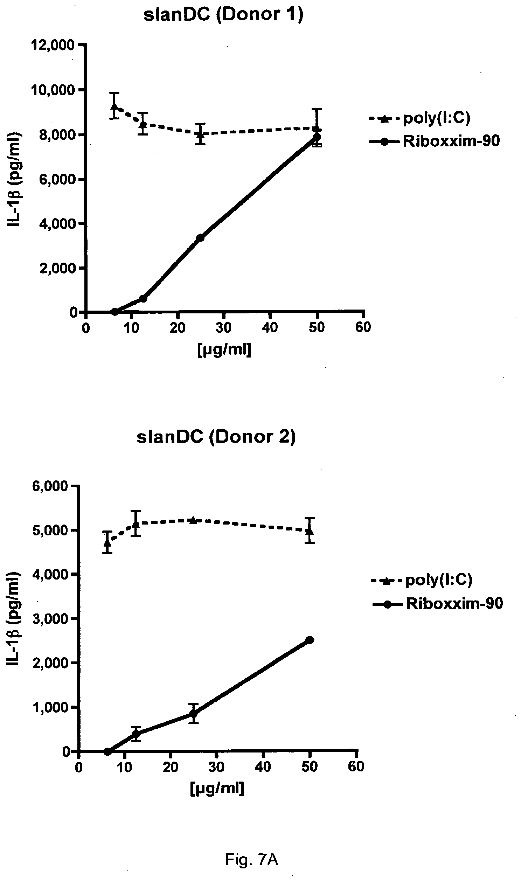

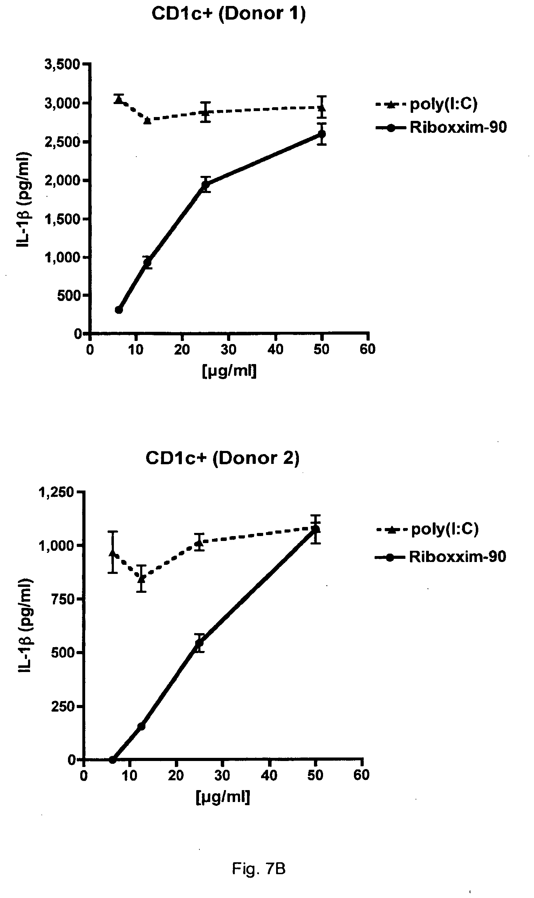

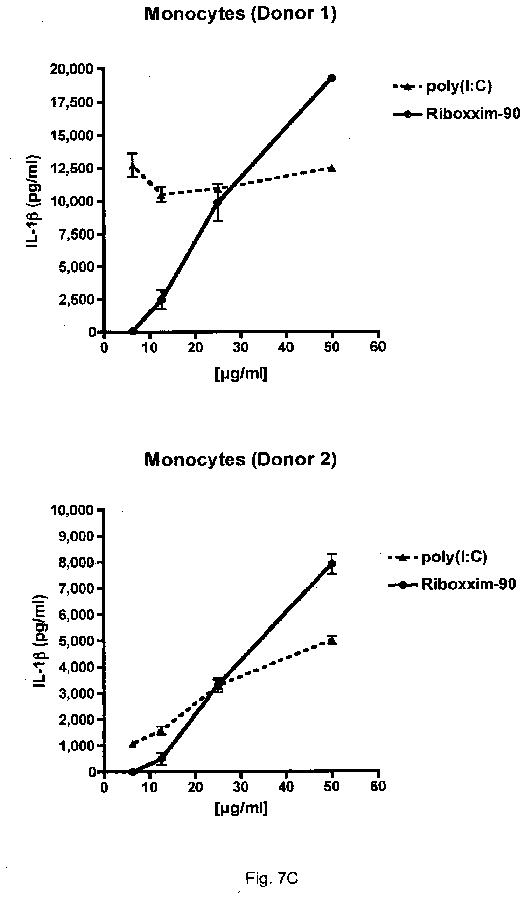

Fig. 7 shows diagrams of the concentration of IL-1β in the cell culture supernatant of slan dendritic cells (Fig. 7A), myeloid dendritic cells (CD1c+, Fig. 7B) and monocytes (Fig. 7C) indicating a dose-dependent secretion of IL-1β after stimulation with Riboxxim®-90 in contrast to a dose-independent, generally high secretion of IL-1β after stimulation with poly(I:C). The cells were stimulated with 6.25 µg/ml, 12.5 µg/ml, 25 µg/ml, and 50 µg/ml of the respective nucleic acid.

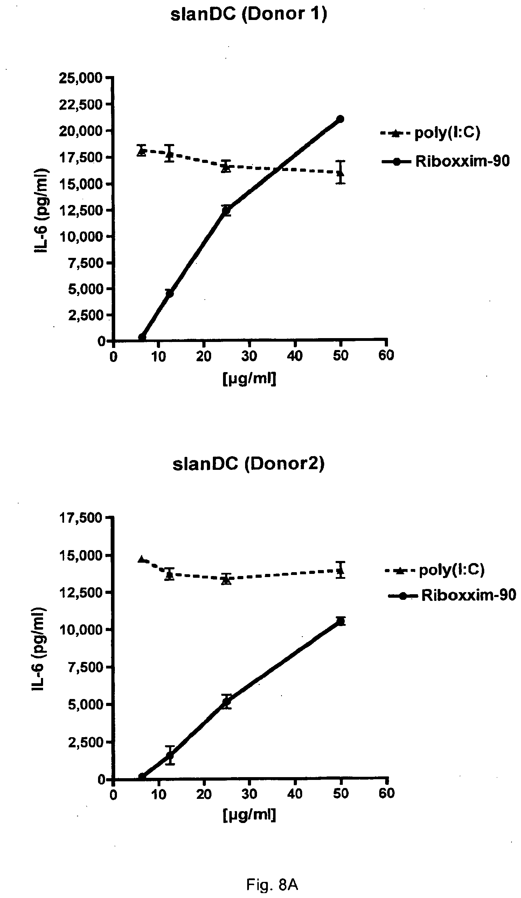

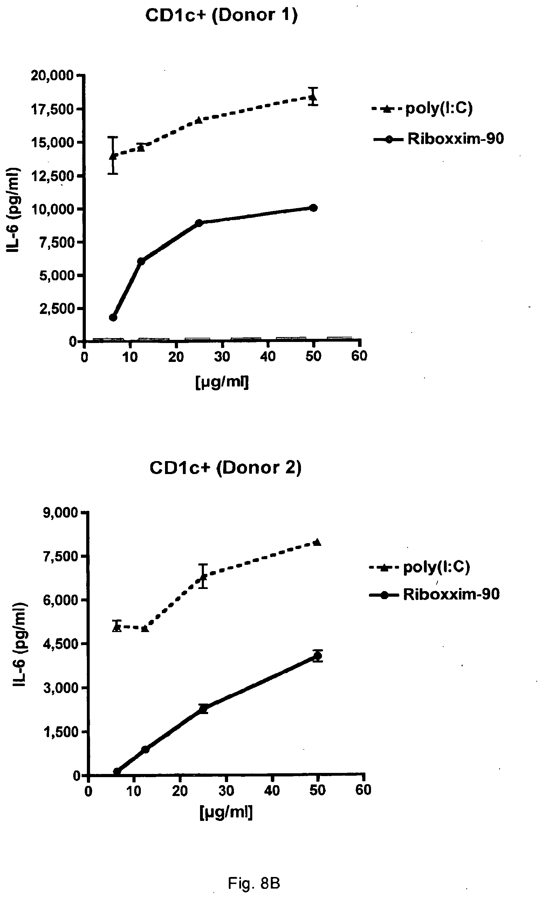

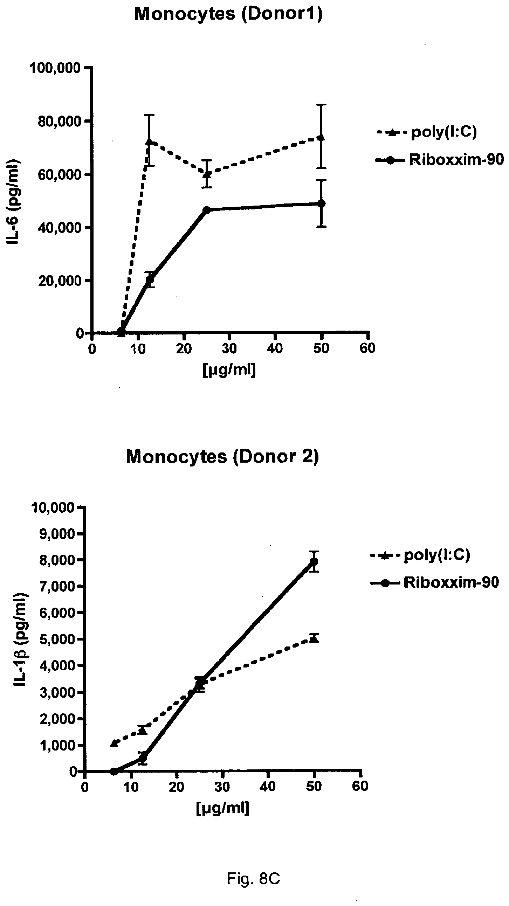

Fig. 8 shows diagrams of the concentration of IL-6 in the cell culture supernatant of slan dendritic cells (Fig. 8A), myeloid dendritic cells (CD1c+, Fig. 8B) and monocytes (Fig. 8C) indicating a dose-dependent secretion of IL-6 after stimulation with Riboxxim®-90 in comparison to the secretion of IL-6 after stimulation with poly(I:C). The cells were stimulated with 6.25 µg/ml, 12.5 µg/ml, 25 µg/ml, and 50 µg/ml of the respective nucleic acid.

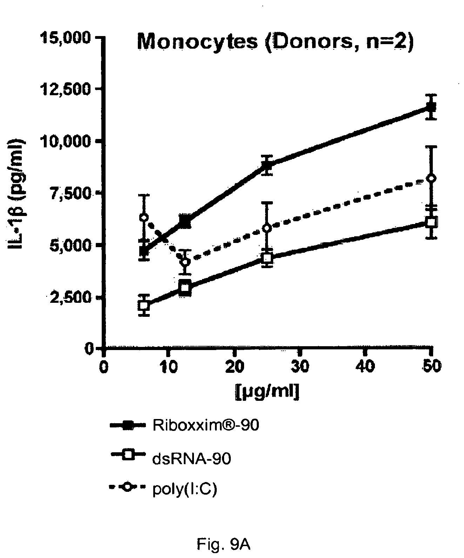

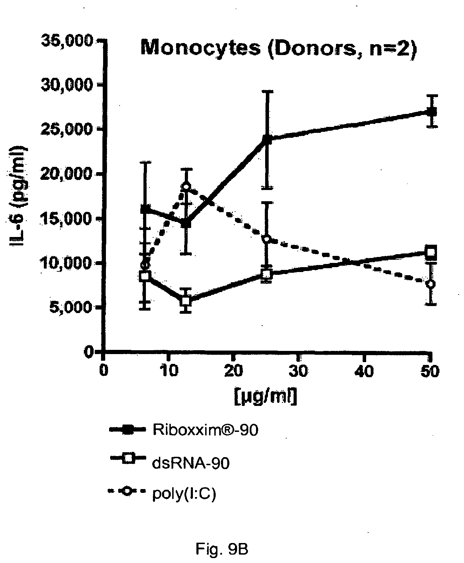

Fig. 9 shows diagrams of the concentration of IL1β (Fig. 9A) and IL-6 (Fig. 9B), respectively, in the cell culture supernatant of monocytes isolated from two different donors indicating a dose-dependent secretion of the respective interleukin after stimulation with triphosphated Riboxxim®-90 or not triphosphated dsRNA90 in comparison to the stimulation with poly(I:C). The mean values +/- SEM of at least three independent measurements per blood donor are shown.

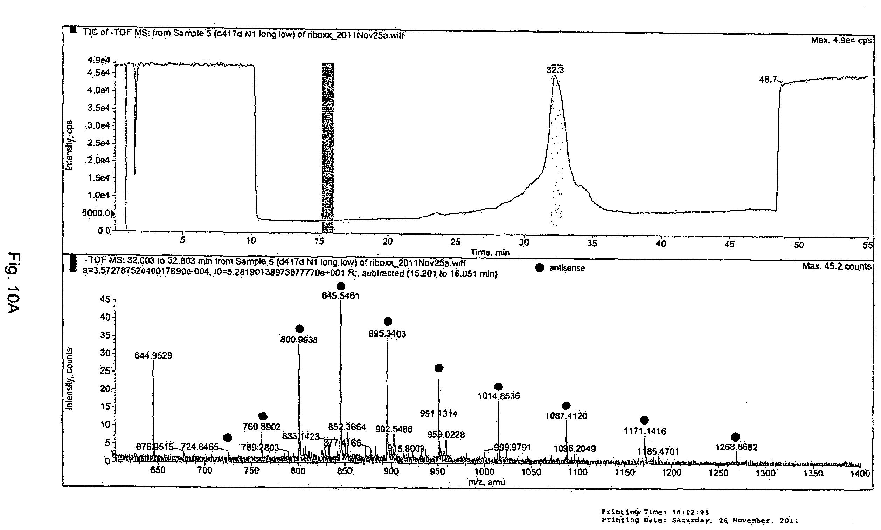

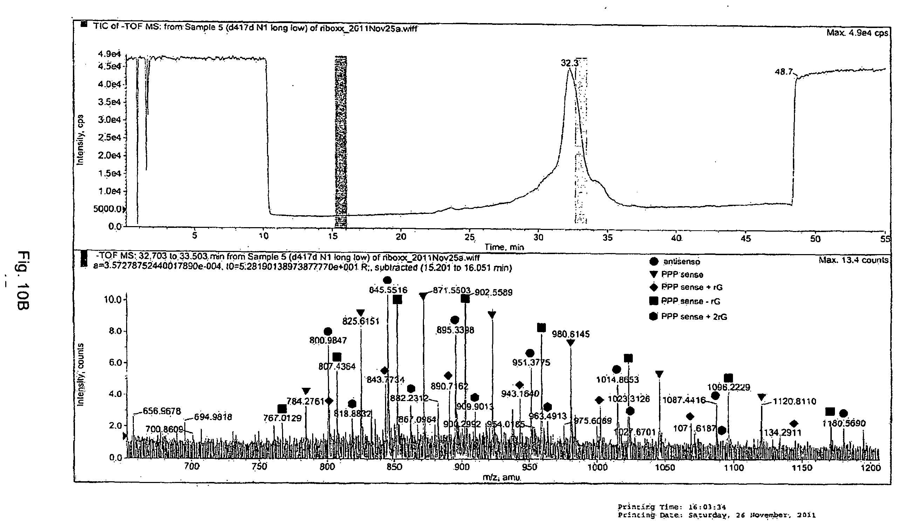

Fig. 10 shows the results of an electrospray ionization mass spectrometry of a double-stranded RNA-molecule bearing a triphosphate moiety at one 5' end, Riboxxim®-48; The MS-analysis of the antisense strand, shown in Figure 10A, corresponds to the calculated mass of the strand with the following sequence:
   5'-CCCCCUAAGCACGAAGCUCAGAGUUAAGCACGAAGCUCAGAGUCCCCC-3' (SEQ ID NO: 8). The different ionization states are depicted. The MS-analysis of the sense strand, shown in Figure 10B, corresponds to the calculated mass of the strand with the following sequence:
      ppp-5'-GGGGGACUCUGAGCUUCGUGCUUAACUCUGAGCUUCGUGCUUAGGGGG-3' (SEQ ID NO: 9).

This strand bears a triphosphate moiety at its 5'-end. The different ionization states are shown.

The present invention is further illustrated by the following non-limiting examples.

### EXAMPLES:

### Example 1: Serum stability of dsRNAs of the invention

A reaction with a total volume of 50 µl contained 0.08 nmole double-stranded RNA species (Riboxxim®-1, see Fig. 1A; Riboxxim®-90, see Fig. 1B; control dsRNA of 50 bp having an arbitrary sequence, see Fig. 1B) and 80% fetal calf serum (FCS). The reaction was incubated at 37°C. At the time points indicated in Fig. 1A and 1B, respectively (15 min; 1h; 2h; 4h; 6h; 24h; 48h; 72h) 5 µl of the reaction was withdrawn at each time point and frozen at -80°C. As a further control, the RNA was spiked in the reaction at the indicated time points (15 min and 1 h) in Fig. 1B. The reaction products at the above time points were separated on a native 20% polyacrylamide gel and the bands visualized by UV transillumination after ethidium bromide staining.

### Example 2: Resistance of dsRNAs of the invention to human DICER

The dsRNA molecules indicated in Fig. 2A were incubated in a total volume of 15 µl containing 250 ng of the respective double-strained RNA (Riboxxim®-1, Riboxxim®-2 or Riboxxim®-3), 2 µl recombinant human DICER enzyme (1U), 4 µl DICER reaction Buffer, 0.5 µl of 50 mM MgCl₂, 1 µl of 10 mM ATP, and RNAse-DNAse-free water to a final volume of 15 µl. The reaction was incubated at 37°C for 12 hours, then separated on a native 20% polyacrylamide gel and visualized through UV transillumination after ethidium bromide staining.

Figure 2B shows the results of incubation with the human DICER complex. Neither of the ribonucleic acids of the invention can be processed by DICER.

### Example 3: Secretion of IL-6 after stimulation with double-stranded RNA molecule according to the invention having a triphosphate moiety at one 5' end

Cells were isolated from the blood of two healthy donors. All cell populations were sorted on two columns via the autoMACS system according to the recommendation of the manufacturer (Miltenyi Biotech GmbH, Bergisch Gladbach, Germany). A detailed protocol for the isolation of 6-Sulfo LacNAc dendritic cells (slanDC) is described in Schaekel, K. et al. (1998); Eur. J. Immunol. 28, (p. 4084-4093). Briefly, myeloid dendritic cells (CD1c+) were isolated after purifying the slanDC; monocytes were isolated by a negative selection strategy. Isolated cells were grown in RPMI 1640 medium containing 10% human AB serum (CCPRO, Neustadt, Germany), 2 mM L-glutamine, 1% nonessential amino acids, 100 U/ml penicillin, and 100 mg/ml streptomycin (Biochrom AG, Berlin, Germany). Cells were then seeded in 96-well plates at a density of 25,000 cells/well in RPMI-1640 with 10% human AB serum. Subpopulations of dendritic cells or monocytes were stimulated with 50 µg/ml Riboxxim®-48 or Riboxxim®-90 for 24 to 72 hours. The concentration of secreted IL-6 in the culture supernatant was assessed using ELISA.

Figure 5 shows the concentration of secreted IL-6 in the cell culture supernatant of dendritic cells and monocytes after incubation with Riboxxim®-48 (Fig. 5A) and Riboxxim®-90 (Fig. 5B), respectively. Both nucleic acids strongly stimulate the secretion of IL-6 in all cell types. The data shown correspond to the mean values of three independent experiments with the respective standard error of the mean.

### Example 4: Secretion of IL-6 in response to a double-stranded RNA molecule according to the invention

Myeloid dendritic cells (CD1c+) and monocytes were isolated, sorted and grown as described in example 3. Cells were then seeded in 96-well plates at a density of 25,000 cells/well in RPMI-1640 with 10% human AB serum and incubated with 50 µg/ml double-stranded RNA (dsRNA-90) for 24 to 72 hours. The concentration of secreted IL-6 in the culture supernatant was analysed using ELISA.

Figure 6 shows the concentration of IL-6 secreted by dendritic cells and monocytes after stimulation with double-stranded RNA without a triphosphate moiety at the 5' end. The data shown correspond to the mean values of three independent experiments with the respective standard error of the mean.

### Example 5: Secretion of IL-1β after stimulation with double-stranded RNA according to the invention bearing a triphosphate moiety at one 5' end is dose-dependent

Dendritic cells (slan DC and CD1c+) and monocytes were isolated, sorted and grown as described in example 3. Cells were then seeded in 96-well plates at a density of 25,000 cells/well in RPMI-1640 with 10% human AB serum and incubated with 6.25 µg/ml, 12.5 µg/ml, 25 µg/ml, or 50 µg/ml Riboxxim®-90 or poly(I:C) for 24 to 72 hours. The concentration of secreted IL-1ß in the culture supernatant was assessed using ELISA.

Figure 7 shows the concentration of IL-1β secreted by slanDC (Fig. 7A), CD1c+ (Fig. 7B) and monocytes (Fig. 7C) after stimulation with increasing amounts of Riboxxim®-90 or poly(I:C). It is demonstrated that the secretion of IL-1β in response to Riboxxim®-90 is dose-dependent in all three cell types, whereas poly(I:C) triggers a dose-independent and generally high secretion of IL-1β, in particular in dendritic cells. The data shown correspond to the mean values of three independent experiments with the respective standard error of the mean.

### Example 6: Secretion of IL-6 after stimulation with double-stranded RNA according to the invention bearing a triphosphate moiety at one 5' end is dose-dependent

Dendritic cells (slan DC and CD1c+) and monocytes were isolated, sorted and grown as described in example 3. Cells were then seeded in 96-well plates at a density of 25,000 cells/well in RPMI-1640 with 10% human AB serum and incubated with 6,25 µg/ml, 12,5 µg/ml, 25 µg/ml or 50 µg/ml Riboxxim®-90 or poly(I:C) for 24 to 72 hours. The concentration of secreted IL-6 in the culture supernatant was assessed using ELISA.

Figure 8 shows the concentration of IL-6 secreted by slanDC (Fig. 8A), CD1c+ (Fig. 8B) and monocytes (Fig. 8C) after stimulation with increasing amounts of Riboxxim®-90 or poly(I:C). It is demonstrated that the secretion of IL-6 in response to Riboxxim®-90 is dose-dependent in all three cell types, whereas poly(I:C) triggers a dose-independent and generally high secretion of IL-6, in particular in dendritic cells. The data shown correspond to the mean values of three independent experiments with the respective standard error of the mean.

### Example 7: Comparison of IL-1 and IL-6 secretion after stimulation with double-stranded RNA according to the invention bearing or not bearing a triphosphate moiety at one 5'-end

Monocytes from two different healthy donors were isolated, sorted and grown as described in example 3. Cells were then seeded in 96-well plates at a density of 25,000 cells/well in RPMI-1640 with 10% human AB serum and incubated with 6,25 µg/ml, 12,5 µg/ml, 25 µg/ml or 50 µg/ml dsRNA-90, Riboxxim®-90 or poly(I:C), respectively, for 24 to 72 hours. The concentrations of secreted of IL-1β and IL-6 in the culture supernatant were assessed using ELISA. The results are shown in Fig. 9A (IL-1β) and Fig. 9B (IL-6).

Both dsRNAs of the invention show a dose-dependent secretion of IL-1β and IL-6. The stimulation is higher for the triphosphated construct Riboxxim®-90. Poly(I:C) does not result in a dose-dependent secretion of IL-6.

The examples described above show that double-stranded RNA-molecules according to the invention trigger the secretion of cytokines such as IL-6 and IL-1β in dendritic cells and monocytes. Moreover, dsRNA of the invention containing a free 5'-triphosphate group results in a strictly dose-dependent cytokine secretion. The capability of triggering a dose-dependent immune-response makes the double-stranded RNA-molecules according to the invention highly suitable to be utilized as RNA drugs for immunostimulation or adjuvants.

## Claims

1. A double-stranded ribonucleic acid of at least 45 bp having the following structure:
5'-(G)₅(N)_{y}(G)₅-3'
3'-(C)₅(N)_{y}(C)₅-5'
or
5'-(C)₅(N)_{y}(C)₅-3'
3'-(G)₅(N)_{y}(G)₅-5'
or
5'-(G)₅(N)_{y}(C)₅-3'
3'-(C)₅(N)_{y}(G)₅-5'
or
5'-(C)₅(N)_{y}(G)₅-3'
3'-(G)₅(N)_{y}(C)₅-5'
wherein N is any ribonucleotide with the proviso that the sequences of both strands are essentially complementary; wherein y is an integer of from 35 to 200, preferably from 35 to 150, more preferably from 35 to 100, particularly preferred from 35 to 80, even more preferred from 35 to 40,-y in one strand is equal to the y in the other strand;
and wherein the nucleotide sequence N between the last 6 to 20 bp at each end of the strands of the double-stranded structure is heteropolymeric, wherein ribonucleic acids having the following sequences are excluded:
GGGUUUUUUUUUUUUUUUGGGUUUUUUUUUUUUUUUGGGUUUUUUUUUUUUUUUGGG (SEQ ID NO: 3)
GGGUUUGGGUUUGGGUUUGGGUUUGGGUUUGGGUUUGGGUUUGGGUUUGGG (SEQ ID NO: 4)
CCCUUUUUUUUUUUUUUUCCCUUUUUUUUUUUUUUUCCCUUUUUUUUUUUUUUUCCC (SEQ ID NO: 5)
CCCUUUCCCUUUCCCUUUCCCUUUCCCUUUCCCUUUCCCUUUCCCUUUCCC (SEQ ID NO: 6).

2. Ribonucleic acid of claim 1 comprising at least one single-stranded overhang.

3. Ribonucleic acid according to any one of the preceding claims wherein the G/C content of said double-stranded ribonucleic acid is at least 45 %.

4. Ribonucleic acid of claim 3 wherein the G/C content is from 45 to 70%.

5. Ribonucleic acid of claim 4 wherein the C/C content is from 48 to 60 %.

6. Ribonucleic acid according to any one of claims 3 to 5 wherein the Tm of said double-stranded structure is from 68 to 80 °C.

7. Ribonucleic acid of claim 1 wherein the strands are one of the following sequence pairs:
5'-CCCCCUAAGCACGAAGCUCAGAGUUAAGCACGAAGCUCAGAGUCCCCC-3' (SEQ ID NO: 8)
5'-GGGGGACUCUGAGCUUCGUGCUUAACUCUGAGCUUCGUGCUUAGGGGG-3' (SEQ ID NO: 9)
5'-CCCCCGAACGAAUUUAUAAGUGGGAACGAAUUUAUAAGUGGCCCCC-3' (SEQ ID NO: 10)
5'-GGGGGCCACUUAUAAAUUCGUUCCCACUUAUAAAUUCGUUCGGGGG-3' (SEQ ID NO: 11)
5'-CCCCCACAACAUUCAUAUAGCUGACAACAUUCAUAUAGCUGCCCCC-3' (SEQ ID NO: 12)
5'-GGGGGCAGCUAUAUGAAUGUUGUCAGCUAUAUGAAUGUUGUGGGGG-3' (SEQ ID NO: 13)

8. Ribonucleic acid according to any one of the preceding claims comprising a free triphosphate group at the 5'-end of at least one strand.

9. Ribonucleic acid of claim 8 wherein the strands are selected from the following sequence pairs:
5'-CCCCCUAAGCACGAAGCUCAGAGUUAAGCACGAAGCUCAGAGUCCCCC-3' (SEQ ID NO: 8)
ppp-5'-GGGGGACUCUGAGCUUCGUGCUUAACUCUGAGCUUCGUGCUUAGGGGG-3 (SEQ ID NO: 9) wherein ppp denotes a free triphosphate group.

10. A pharmaceutical composition comprising at least one ribonucleic acid according to any one of the preceding claims in combination with at least one pharmaceutically acceptable carrier.

11. Pharmaceutical composition of claim 10 comprising a vaccine.

12. The ribonucleic acid according to any one of claims 1 to 9 for use as a medicament.

13. The ribonucleic acid according to any one of claims 1 to 9 for use in immunostimulation.

14. The ribonucleic acid according to any one of claims 1 to 9 for use as an agonist of Toll-like receptor 3 (TLR-3) in the treatment of infectious diseases and tumours.

15. The ribonucleic acid of claim 8 or 9 for use as an agonist of TLR-3 and RIG-I in the treatment of infectious diseases and tumours.

16. A method of producing the ribonucleic acid according to any one of claims 1 to 9 comprising the step of:
synthesizing a first ribonucleic acid strand and a second ribonucleic acid strand and annealing the two strands wherein the strands have the appropriate sequences so as to form the double-stranded structure as defined in any one of claims 1 to 9.

## Patentansprüche

1. Doppelsträngige Ribonukleinsäure mit mindestens 45 Bp, die folgende Struktur aufweist:
5'-(G)₅(N)_{y}(G)₅-3'
3'-(C)₅(N)_{y}(C)₅-5'
oder
5'-(C)₅(N)_{y}(C)₅-3'
3'-(G)₅(N)_{y}(G)₅-5'
oder
5'-(G)₅(N)_{y}(C)₅-3'
3'-(C)₅(N)_{y}(G)₅-5'
oder
5'-(C)₅(N)_{y}(G)₅-3'
3'-(G)₅(N)_{y}(C)₅-5'
wobei N ein beliebiges Ribonukleotid mit der Maßgabe ist, dass die Sequenzen in beiden Strängen im Wesentlichen komplementär sind; jedes y eine ganze Zahl von 35 bis 200, vorzugsweise von 35 bis 150, mehr bevorzugt von 35 bis 100, insbesondere bevorzugt von 35 bis 80, noch mehr bevorzugt von 35 bis 40 ist, y in einem Strang gleich dem y in dem anderen Strang ist;
und wobei die Nukleotidsequenz N zwischen den letzten 6 bis 20 Bp an jedem Ende der Stränge der doppelsträngigen Struktur heteropolymer ist, wobei Ribonukleinsäuren mit den folgenden Sequenzen ausgeschlossen sind:
GGGUUUUUUUUUUUUUUUGGGUUUUUUUUUUUUUUUGGGUUUUUUUUUUUUUUUGGG (SEQ ID NO: 3)
GGGUUUGGGUUUGGGUUUGGGUUUGGGUUUGGGUUUGGGUUUGGGUUUGGG (SEQ ID NO: 4)
CCCUUUUUUUUUUUUUUUCCCUUUUUUUUUUUUUUUCCCUUUUUUUUUUUUUUUCCC (SEQ ID NO: 5)
CCCUUUCCCUUUCCCUUUCCCUUUCCCUUUCCCUUUCCCUUUCCCUUUCCC (SEQ ID NO: 6) .

2. Ribonukleinsäure nach Anspruch 1, die mindestens einen einzelsträngigen Überhang umfasst.

3. Ribonukleinsäure nach einem der vorherigen Ansprüche, wobei der G/C-Gehalt der doppelsträngigen Ribonukleinsäure mindestens 45 % beträgt.

4. Ribonukleinsäure nach Anspruch 3, wobei der G/C-Gehalt 45 bis 70 % beträgt.

5. Ribonukleinsäure nach Anspruch 4, wobei der G/C-Gehalt 48 bis 60 % beträgt.

6. Ribonukleinsäure nach einem der Ansprüche 3 bis 5, wobei der Tm der doppelsträngigen Struktur 68 bis 80 °C beträgt.

7. Ribonukleinsäure nach Anspruch 1, wobei die Stränge eines der folgenden Sequenzpaare sind:
5'-CCCCCUAAGCACGAAGCUCAGAGUUAAGCACGAAGCUCAGAGUCCCCC-3' (SEQ ID NO: 8)
5'-GGGGGACUCUGAGCUUCGUGCUUAACUCUGAGCUUCGUGCUUAGGGGG-3' (SEQ ID NO: 9)
5'-CCCCCGAACGAAUUUAUAAGUGGGAACGAAUUUAUAAGUGGCCCCC-3' (SEQ ID NO: 10)
5'-GGGGGCCACUUAUAAAUUCGUUCCCACUUAUAAAUUCGUUCGGGGG-3' (SEQ ID NO: 11)
5'-CCCCCACAACAUUCAUAUAGCUGACAACAUUCAUAUAGCUGCCCCC-3' (SEQ ID NO: 12)
5'-GGGGGCAGCUAUAUGAAUGUUGUCAGCUAUAUGAAUGUUGUGGGGG-3' (SEQ ID NO: 13)

8. Ribonukleinsäure nach einem der vorhergehenden Ansprüche, die eine freie Triphosphatgruppe am 5'-Ende mindestens eines Strangs umfasst.

9. Ribonukleinsäure nach Anspruch 8, wobei die Stränge aus den folgenden Sequenzpaaren ausgewählt sind:
5'-CCCCCUAAGCACGAAGCUCAGAGUUAAGCACGAAGCUCAGAGUCCCCC-3' (SEQ ID NO: 8)
ppp-5'-GGGGGACUCUGAGCUUCGUGCUUAACUCUGAGCUUCGUGCUUAGGGGG-3' (SEQ ID NO: 9) wobei ppp eine freie Triphosphatgruppe bedeutet.

10. Pharmazeutische Zusammensetzung, die mindestens eine Ribonukleinsäure nach einem der vorhergehenden Ansprüche in Kombination mit mindestens einem pharmazeutisch verträglichen Träger umfasst.

11. Pharmazeutische Zusammensetzung nach Anspruch 10, die ein Vakzin umfasst.

12. Ribonukleinsäure nach einem der Ansprüche 1 bis 9 zur Verwendung als Medikament.

13. Ribonukleinsäure nach einem der Ansprüche 1 bis 9 zur Verwendung in der Immunstimulation.

14. Ribonukleinsäure nach einem der Ansprüche 1 bis 9 zur Verwendung als Toll-like-Rezeptor-3 (TLR-3)-Agonist in der Behandlung von infektiösen Erkrankungen und Tumoren.

15. Ribonukleinsäure nach Anspruch 8 oder 9 zur Verwendung als TLR-3- und RIG-I-Agonist in der Behandlung von infektiösen Erkrankungen und Tumoren.

16. Verfahren zur Herstellung der Ribonukleinsäure nach einem der Ansprüche 1 bis 9, das den Schritt
Synthetisieren eines ersten Ribonukleinsäurestrangs und eines zweiten Ribonukleinsäurestrangs und Hybridisieren der beiden Stränge umfasst, wobei die Stränge die geeigneten Sequenzen aufweisen, um die doppelsträngige Struktur gemäß der Definition in einem der Ansprüche 1 bis 9 auszubilden.

## Revendications

1. Acide ribonucléique double brin d'au moins 45 pb présentant la structure suivante :
5'-(G)₅(N)_{y}(G)₅-3'
3'-(C)₅(N)_{y}(C)₅-5'
ou
5'-(C)₅(N)_{y}(C)₅-3'
3'-(G)₅(N)_{y}(G)₅-5'
ou
5'-(G)₅(N)_{y}(C)₅-3'
3'-(C)₅(N)_{y}(G)₅-5'
ou
5'(C)₅(N)_{y}(G)₅-3'
3'-(G)₅(N)_{y}(C)₅-5'
dans lequel N est tout ribonucléotide à condition que les séquences des deux brins soient essentiellement complémentaires ; chaque y est un nombre entier de 35 à 200, de préférence de 35 à 150, plus préférablement de 35 à 100, de manière particulièrement préférée de 35 à 80, encore plus préférablement de 35 à 40 ; y dans un brin soit égal à l'y dans l'autre brin;
et dans lequel la séquence nucléotidique N entre les 6 à 20 dernières pb à chaque extrémité des brins de la structure double brin est hétéropolymère, dans lequel les acides ribonucléiques ayant les séquences suivantes sont exclus :
GGGUUUUUUUUUUUUUUUGGGUUUUUUUXJUUUUUUUGGGUUUUUUUUUUUUUUUGGG (SEQ ID N° : 3)
GGGUUUGGGUUUGGGUUUGGGUUUGGGUUUGGGUUUGGGUUUGGGUUUGGG (SEQ ID N° : 4)
CCCUUUUUUUUTJUUUUUUCCCUUUUUUUUUUUUUUUCCCUUUUUUUUUUUUUUUCCC (SEQ ID N° : 5)
CCCUUUCCCUUUCCCUUUCCCUUUCCCUUUCCCUUUCCCUUUCCCUUUCCC (SEQ ID N° : 6).

2. Acide ribonucléique selon la revendication 1 comprenant au moins un débordement simple brin.

3. Acide ribonucléique selon l'une quelconque des revendications précédentes dans lequel la teneur en G/C dudit acide ribonucléique double brin est d'au moins 45 %.

4. Acide ribonucléique selon la revendication 3 dans lequel la teneur en G/C est de 45 à 70 %.

5. Acide ribonucléique selon la revendication 4 dans lequel la teneur en C/C est de 48 à 60 %.

6. Acide ribonucléique selon l'une quelconque des revendications 3 à 5 dans lequel la Tm de ladite structure double brin est de 68 à 80 °C.

7. Acide ribonucléique selon la revendication 1 dans lequel les brins sont l'une des paires de séquences suivantes :
5'-CCCCCUAAGCACGAAGCUCAGAGILTUAAGCACGAAGCUCAGAGUCCCCC-3' (SEQ ID N° : 8)
5'-GGGGGACUCUGAGCUUCGUGCUUAACUCUGAGCUUCGUGCUUAGGGGG-3' (SEQ ID N° : 9)
5'-CCCCCGAACGAAUUUAUAAGUGGGAACGAAUUUAUAAGUGGCCCCC- 3' (SEQ ID N° : 10)
5'-GGGGGCCACUUAUAAAUUCGUUCCCACUUAUAAAUUCGUUCGGGGG-3' (SEQ ID N° : 11)
5'-CCCCCACAACAUUCAUAUAGCUGACAACAUUCAUAUAGCUGCCCCC-3' (SEQ ID N° : 12)
5'-GGGGGCAGCUAUAUGAAUGUUGUCAGCUAUAUGAAUGUUGUGGGGG- 3' (SEQ ID N° : 13)

8. Acide ribonucléique selon l'une quelconque des revendications précédentes comprenant un groupe triphosphate libre à l'extrémité 5' d'au moins un brin.

9. Acide ribonucléique selon la revendication 8 dans lequel les brins sont choisis parmi les paires de séquences suivantes :
5'-CCCCCUAAGCACGAAGCUCAGAGUUAAGCACGAAGCUCAGAGUCCCCC-3' (SEQ ID N° : 8)
ppp-5' - GGGGGACUCUGAGCUUCGUGCUUAACUCUGAGCUUCGUGCUUAGGGGG-3' (SEQ ID N°9) dans lequel ppp désigne un groupe triphosphate libre.

10. Composition pharmaceutique comprenant au moins un acide ribonucléique selon l'une quelconque des revendications précédentes en association avec au moins un vecteur pharmaceutiquement acceptable.

11. Composition pharmaceutique selon la revendication 10 comprenant un vaccin.

12. Acide ribonucléique selon l'une quelconque des revendications 1 à 9 pour utilisation en tant que médicament.

13. Acide ribonucléique selon l'une quelconque des revendications 1 à 9 pour utilisation dans l'immunostimulation.

14. Acide ribonucléique selon l'une quelconque des revendications 1 à 9 pour utilisation en tant qu'agoniste du récepteur Toll-like 3 (TLR-3) dans le traitement des maladies infectieuses et des tumeurs.

15. Acide ribonucléique selon la revendication 8 ou 9 pour utilisation en tant qu'agoniste du TLR-3 et du RIG-I dans le traitement des maladies infectieuses et des tumeurs.

16. Procédé de production de l'acide ribonucléique selon l'une quelconque des revendications 1 à 9 comprenant l'étape de :
synthèse d'un premier brin d'acide ribonucléique et d'un second brin d'acide ribonucléique et liaison des deux brins dans lequel les brins ont les séquences appropriées pour former la structure double brin selon l'une quelconque des revendications 1 à 9.
